# FASCICULE DE BREVET EUROPEEN

(11) **EP 1 336 606 B1**
(45) Date de publication et mention de la délivrance du brevet: **27.12.2006**
(21) Numéro de dépôt: 03290292.6
(22) Date de dépôt: 05.02.2003
(51) Int. Cl.: C07D 213/74, C07D 401/04, A61K 8/49, A61Q 5/10

(54) **Nouveaux coupleurs 6-alcoxy-2,3-diaminopyridine utiles pour la teinture des fibres kératiniques**
6-Alkoxy-2,3-Diaminopyridin-Farbstoffkuppler zur Färbung von Keratinfasern
6-Alkoxy-2,3-diaminopyridine couplers for dyeing keratin fibers

(30) Priorité: 12.02.2002 FR 0201710
(43) Date de publication de la demande: 20.08.2003
(73) Titulaire: L'ORÉAL, 75008 Paris (FR)
(72) Inventeur: Vidal, Laurent, 75013 Paris (FR); Fadli, Aziz, 77500 Chelles (FR)
(74) Mandataire: Fevrier, Murielle Françoise E.

(56) Documents cités:
- WO-A-00/43392
- US-A- 4 784 667
- PRUNIER, HERVE ET AL: "Novel and Selective Partial Agonists of 5-HT3 Receptors. 2. Synthesis and Biological Evaluation of Piperazinopyridopyrrolopyrazines, Piperazinopyrroloquinoxalines, and Piperazinopyridopyrroloquinoxalines" JOURNAL OF MEDICINAL CHEMISTRY., vol. 40, no. 12, 1997, pages 1808-1819, XP002218035 AMERICAN CHEMICAL SOCIETY. WASHINGTON., US ISSN: 0022-2623

## Description

L'invention a pour objet une composition tinctoriale utile pour la teinture des fibres kératiniques contenant au moins une base d'oxydation et au moins un coupleur du type 6-alcoxy-2,3-diaminopyridine, l'utilisation de cette composition pour la teinture des fibres kératiniques ainsi que le procédé de teinture mettant en oeuvre cette composition. L'invention a aussi pour objet de nouveaux composés 6-alcoxy-2,3-diaminopyridines utiles comme coupleurs.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

On connaît dans le brevet FR 1397551 des compositions tinctoriales contenant des précurseurs de colorant d'oxydation du type dérivé pyridinique tri-substitués, chacun des substituants pouvant être un radical hydroxy, alcoxy, amino, ou NR₁R₂ avec R₁R₂ représentant un H, alkyle, aryle. La coloration est obtenue soit par oxydation à l'air soit par un milieu oxydant contenant de l'eau oxygénée à pH basique. En raison de la forte oxydabilité de ces précurseurs pyridiniques, les teintures obtenues sur cheveux ont tendance à évoluer dans le temps en changeant de couleur, ce qui s'avère particulièrement inesthétique.

Une autre demande de brevet DE 3 233 540 propose de teindre des cheveux par des compositions contenant à titre de coupleur des dérivés de 6-alcoxy-3-aminopyridine substitués en position 2 par un radical NH₂ ou NHR₃ avec R₃ = H, alkyle en C₁-C₄, hydroxy alkyle en C₂-C₄ en association avec des bases d'oxydation classiques. Ces compositions donnent en présence de certaines bases comme la para-phenylène diamine ou la para-toluènediamine des nuances bleues foncées fragiles à la lumière et manquant d'intensité et d'unisson entre la racine et la pointe des cheveux.

La demande de brevet DE 4 115 148 propose des compositions de teintures pour cheveux contenant comme base d'oxydation un dérivé de triamino pyrimidine substitué par un quatrième radical amino ou hydroxy, un méta-aminophénol et un dérivé de 6-méthoxy-3-amino pyridine substitué en position deux par un radical alkyl amino en C₁-C₄. De telles compositions confèrent aux cheveux une coloration noire. Toutefois, les nuances telles que les cuivrés, acajou, rouge, violet bleu ou vertes ne peuvent être obtenues par de telles compositions.

La demande de brevet FR 2 779 952 propose de teindre les cheveux avec des compositions de teinture à base de dérivés de pyridines en tant que coupleur et à base de pyrazolo [1,5-a] pyrimidines en tant que base d'oxydation. Cependant, la coloration obtenue par de telles compositions ne permet pas d'obtenir une homogénéité (ou un unisson) de la couleur de la racine à la pointe des cheveux. De plus, elle manque d'intensité et de chromaticité.

La demande EP 728 464 propose de teindre des cheveux avec des compositions de teinture à base de 4,5 ou 3,4 diaminopyrazoles comme bases d'oxydation et de dérivés de pyridines comme coupleurs. Cette solution permet d'obtenir des nuances généralement cuivré-acajou, mais celles-ci manquent d'intensité et de ténacité face aux agents extérieurs tels que le shampooing, la lumière et la sueur et la chromaticité.

La demande de brevet DE 199 36442 propose une composition de teinture pour teindre les cheveux humains contenant un dérivé de para-aminophénol comme base d'oxydation avec au moins un coupleur dérivé de méthylaminophénol et/ou un coupleur dérivé de 2-aminopyridine substitué en 3 et 6 par un radical H, amino, hydroxy ou alcoxy. Une telle composition permet uniquement de teindre les cheveux ayant naturellement une couleur châtain. De plus, les teintures résultantes manquent notablement de brillance et de chromaticité.

Le but de la présente invention est de fournir de nouvelles compositions tinctoriales pour la teinture de fibres kératiniques ne présentant pas les inconvénients de celles de la technique antérieure. En particulier, le but de la présente invention est de fournir des compositions qui présentent des teintures puissantes, uniformes entre la racine et la pointe des cheveux, ayant une bonne chromaticité, peu sélectives et particulièrement résistantes, tout en étant capables d'engendrer des colorations intenses dans des nuances variées, en particulier dans les nuances fondamentales.

Ce but est atteint avec la présente invention qui a pour objet une composition tinctoriale comprenant, dans un milieu de teinture approprié, au moins une base d'oxydation et au moins un coupleur 6-alcoxy-2,3-diaminopyridine de formule (I) ou les sels d'addition correspondants : 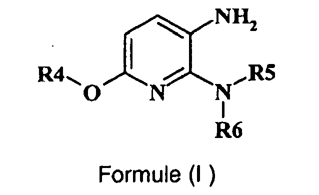 dans laquelle
- R₄ représente un radical alkyle en C₁-C₄ éventuellement substitué par un ou plusieurs radicaux choisis parmi les radicaux hydroxyle, alcoxy en C₁-C₂, NR₇R₈ où R₇ et R₈ sont choisis parmi l'hydrogène, les radicaux alkyle en C₁-C₄, (poly)hydroxyalkyle en C₂-C₆, (poly)aminoalkyle en C₂-C₆, amino-hydroxyalkyle en C₂-C₆ ;
- R₅ et R₆ forment avec l'atome d'azote avec lequel ils sont attachés un cycle comportant 5 à 8 chaînons dont un ou plusieurs atomes de carbone du cycle carboné peuvent être remplacés par un atome d'oxygène, d'azote éventuellement substitué, de soufre ou par un groupement SO₂, et dont les atomes de carbone peuvent être, indépendamment les uns des autres, substitués ; ledit cycle ne comportant pas de liaison peroxyde, ni de radicaux diazo ou nitroso.

De façon préférentielle, R₅ et R₆ forment avec l'atome d'azote avec lequel ils sont attachés un hétérocycle à 5 à 8 chaînons choisis parmi la pyrrolidine, pipéridine, homopipéridine, pipérazine, homopipérazine, diazépane.

Selon un mode de réalisation particulier, lesdits cycles peuvent être substitués par un ou plusieurs radicaux choisis parmi les radicaux suivants :
(i) alkyle en C₁-C₄ éventuellement substitué par un ou plusieurs radicaux choisis parmi les radicaux hydroxy, amino, (di)alkylamino en C₁-C₂ , carboxy ;
(ii) hydroxy,
(iii) amino,
(iv) (di)alkylamino en C₁-C₂,
(v) carboxy,
(vi) carboxamido,
(vii) sulfonamido.

Selon un mode de réalisation préféré, R₅ et R₆ forment ensemble un hétérocycle choisi parmi la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la 3-acétamidopyrrolidine, la 3-(méthylsulfonylamino)-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, le diazépane, le N-méthyl diazépane, le N-(2-hydroxyéthyl)- diazépane et leurs sels d'addition.

Plus préférentiellement, R₅ et R₆, forment ensemble un hétérocycle choisi parmi la pyrrolidine, la 3-hydroxypyrrolidine, la 3-aminopyrrolidine, la 3-acétamidopyrrolidine, la 3-(méthylsulfonylamino)-pyrrolidine, la proline, la 3-hydroxyproline, la pipéridine, les hydroxypipéridines, l'homopipéridine, le diazépane, le N-méthyl diazépane (appelé aussi N-méthyl homopipérazine), le N-(2-hydroxyéthyl)- diazépane (appelé aussi la N β-hydroxyéthylhomopipérazine) et leurs sels d'addition.

Dans le cadre de la présente invention, on entend par alkyle, des radicaux linéaires ou ramifiés par exemple méthyle, éthyle, n-propyle, iso-propyle, butyle, etc. Un radical alcoxy est un radical alk-O, le radical alkyle ayant la définition donnée ci dessus. Halogène désigne de préférence Cl, Br, I.

Les radicaux (poly)aminoalkyle sont des radicaux alkyles substitués par un ou plusieurs radicaux amino. Les radicaux (poly)hydroxyalkyles sont des radicaux alkyles substitués par un ou plusieurs substituants hydroxy.

A titre d'exemples de composés de formule (I), on peut citer :
- la 6-méthoxy-2-pyrrolidin-1-yl-pyridin-3-ylamine ;
- la 2-(2,5-diméthyl-pyrrolidin-1-yl)-6-méthoxy-pyridin-3- ylamine ;
- la 6-méthoxy-2-morpholin-4-yl-pyridin-3-ylamine ;
- le N-[1-(3-amino-6-méthoxy-pyridin-2-yl)-pyrrolidin-3-yl]-acétamide ;
- la 6'-méthoxy-3,4,5,6-tetrahydro-2H-[1,2']bipyridinyl-3'-ylamine ;
- la 2-(3'-Amino-6'-methoxy-3,4,5,6-tetrahydro-2H-I [1,2']bipyridinyl-2-yl)-éthanol ;
- la 6-méthoxy-2-(4-méthyl-pipérazin-1-yl)-pyridin-3-ylamine ;
- la 6 '-méthoxy-2-méthyl-3, 4,5,6-tetrahydro-2H-[1,2']bipyridinyl-13'-ylamine ;
- la 6'-méthoxy-3-methyl-3,4,5,6-tetrahydro-2H-[1,2']bipyridinyl-3'-ylamine;
- la 2-(3'-Amino-6'-méthoxy-3,4,5,6-tetrahydro-2H-[1,2']-bipyridinyl-2-yl)-éthanol ainsi que leurs sels d'addition.

On utilisera plus particulièrement le composé 6-méthoxy-2-pyrrolidin-1-yl-pyridin-3-ylamine ainsi que ses sels d'addition.

La composition de teinture par oxydation de la présente invention comprend une ou plusieurs bases d'oxydation classiquement utilisées en teinture d'oxydation. A titre d'exemple, ces bases d'oxydation sont choisies parmi les bases d'oxydation, par exemple les paraphénylènediamines, les bis-phénylalkylènediamines, les para-aminophénols, les ortho-aminophénols, les bases hétérocycliques autres que celles décrites précédemment et leur sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation sont en général chacune présentes en quantité comprise entre 0,001 à 10 % en poids environ du poids total de la composition tinctoriale, de préférence entre 0,005 et 6 %.

La composition selon l'invention peut contenir un ou plusieurs coupleurs additionnels conventionnellement utilisés pour la teinture de fibres kératiniques. Parmi ces coupleurs additionnels, on peut notamment citer les métaphénylènediamines, les métaaminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 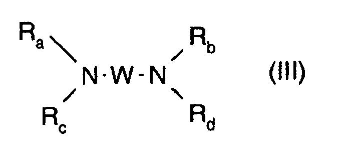 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Selon le procédé de teinture de la présente invention, on applique sur les fibres la composition selon la présente invention, et on révèle la couleur à l'aide d'un agent oxydant. La couleur peut être révélée à pH acide, neutre ou alcalin et l'agent oxydant peut être mélangé à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment pour la composition de l'invention.

Le pH de la composition oxydante renfermant l'agent oxydant est tel qu'après mélange avec la composition tinctoriale, le pH de la composition résultante appliquée sur les fibres kératiniques varie entre 3 et 12 environ, et préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment contient la composition tinctoriale de l'invention définie ci-dessus et un deuxième compartiment contient une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a enfin pour objet les composés 6-alcoxy-2,3-diaminopyridine de formule (I) ainsi que leurs sels d'addition correspondants tels que définis précédemment à l'exception des composés 3-amino 6-méthoxy 2-(4-méthyl 2-propylimidazol-1-yl)-pyridine et 3-amino 6-méthoxy 2-pyrrolo-pyridine.

Ces composés peuvent être synthétisés selon le schéma de synthèse suivant :

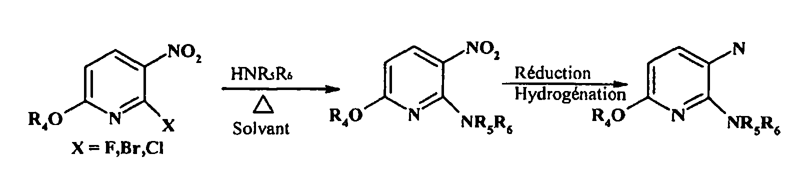

La première étape consiste à faire réagir un dérivé de 6-alcoxy-3-nitro-2-halogéno-pyridine avec une amine de type HNR₅R₆ dans laquelle R₅ et R₆ ont les mêmes significations indiquées ci-dessus dans un solvant polaire de point d'ébullition compris entre 70°C et 180°C. La température de réaction varie selon les dérivés de pyridine et l'amine nucléophile, de 75°C à 140°C. De préférence, on choisira comme solvant, les alcools tels que l'éthanol, isopropanol, le butanol, le pentanol ainsi que l'acide acétique, formique ou le dioxane et la DMF.

La deuxième étape est une réaction de réduction réalisée soit par hydrogénation en catalyse hétérogène, soit par transfert d'hydrogène ou encore par des hydrures métalliques ou encore par le couple acide formique-acide acétique en présence de palladium.

Par exemple, on utilise la méthode largement illustrée dans la littérature d'hydrogénation catalysée par le palladium (0), Pd (II), ou encore par du nickel de Raney ou PtO₂.

La réduction par transfert d'hydrogène faisant réagir du cyclohexene en présence de palladium s'avère également très efficace

Les exemples qui suivent servent à illustrer l'invention sans toutefois présenter un caractère limitatif.

### EXEMPLES DE SYNTHESE :

### Exemple n°1 : 6-méthoxy-2-pyrrolidin-1-yl-pyridin-3-ylamine

### A) Synthèse du 6-méthoxy-3-nitro-2-pyrrolidin-1-yl-pyridine:

Dans un ballon tout équipé, on charge 10g (0.053 mole) de 2-chloro-3-nitro-6-méthoxypyridine, 60ml d'éthanol, 7.54 g (0.1mol) de pyrrolidine.

Le mélange est porté au reflux pendant deux heures sous agitation puis ce milieu réactionnel est versé sous agitation sur un mélange de glace-eau. Le précipité formé est essoré et séché sous vide jusqu'à poids constant.

On obtient 11.6g de poudre jaune (Rdt = 98.3%, pF =82°C)

Analyses en spectrométrie de masse, en spectroscopie de résonance magnétique : conformes

### B) Synthèse du 6-méthoxy-2-pyrrolidin-1-yl-pyridin-3-ylamine

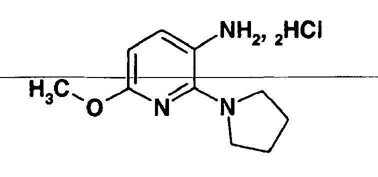

Dans un hydrogénateur, on charge 10g (0.045 mole)de 6-méthoxy-3-nitro-2-pyrrolidin-1-yl-pyridine synthétisé selon le mode opératoire si dessous, 150ml d'éthanol et 2 g de palladium sur charbon.

Le mélange est réduit pendant deux heures sous une pression de 8 Bars puis le catalyseur est éliminé par filtration et le filtrat est acidifié par de l'acide chlorhydrique.

Après dilution à l'éther diisopropylique le précipité formé est essoré et séché sous vide jusqu'à poids constant.

On obtient 11 g de poudre beige, Rdt=92.3%

Analyse en spectrométrie de masse, en spectroscopie de résonance magnétique : conformes

| Analyse CHCINO : | | | | | |
|---|---|---|---|---|---|
| Trouvée : | C 45.11 | H 6.68 | N 15.18 | O 9.86 | CI 24.13 |
| Calculée : | C 45.13 | H 6.44 | N 15.79 | O 6.01 | CI 26.64 |

### Exemple n°2 : 2-(2,5-diméthyl-pyrrolidin-1-yl)-6-méthoxy-pyridin-3-ylamine

### A) Synthèse du 2-(2,5-diméthyl-pyrrolidin-1-yl)-6-méthoxy-3-nitro-pyridine:

Dans un ballon tout équipé, on charge 4g (0.0212 mole) de produit 2-chloro-3-nitro-6-méthoxypyridine, 40ml d'éthanol, 4.2 g (0.042 mol) de 2,5-diméthylpyrrolidine.

Le mélange est porté au reflux pendant deux heures sous agitation puis le mélange est versé sur un mélange de glace eau sous agitation. Le précipité formé est essoré et séché sous vide jusqu'à poids constant.

On obtient 4.93g de poudre jaune

Analyse en spectrométrie de masse, en spectroscopie de résonance magnétique : conformes.

### B) Synthèse du 2-(2,5-diméthyl-pyrrolidin-1-yl)-6-méthoxy-pyridin-3- ylamine

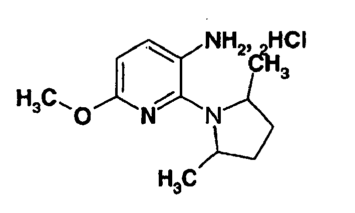

Dans un ballon tout équipé, on charge 4.2g (0.017 mole) de produit 2-(2,5-diméthyl-pyrrolidin-1-yl)-6-méthoxy-3-nitro-pyridine synthétisé selon le mode opératoire si dessous, 40ml d'éthanol, 10ml de cyclohexene et 1.5 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique, le précipité formé est essoré et séché sous vide jusqu'à poids constant.

On obtient 4.28g de poudre Rdt = 89.2%

Analyse en spectrométrie de masse, en spectroscopie de résonance magnétique : conformes.

| Analyse CHClNO : | | | | | |
|---|---|---|---|---|---|
| Trouvée : | C54.05 | H 7.26 | N 15.57 | O 11.66 | Cl 11.68 |
| Calculée : | C 55.92 | H7.82 | N16.3 | O 6.21 | CI 13.75 |

### Exemple n°3 : 6-méthoxy-2-morpholin-4-yl-pyridin-3-ylamine

### A) Synthèse du : 4-(6-méthoxy-3-nitro-pyridin-2-yl)-morpholine

Dans un ballon tout équipé, on charge 5g (0.0265 mole) de produit 2-chloro-3-nitro-6-méthoxypyridine, 50ml d'éthanol, 4.62ml (0.053mol)de morpholine.

Le mélange est porté au reflux pendant deux heures sous agitation puis le mélange est versé sur un mélange de glace eau sous agitation. Le précipité formé est essoré et séché sous vide jusqu'à poids constant.

On obtient 6.32g de poudre jaune.

Analyse en spectrométrie de masse, en spectroscopie de résonance magnétique: conformes.

### B) Synthèse du 6-méthoxy-2-morpholin-4-yl-pyridin-3-ylamine

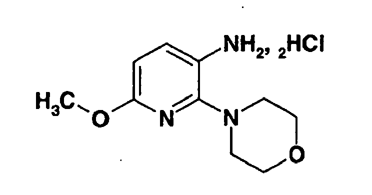

Dans un ballon tout équipé, on charge 6.19g (0.026 mole) de produit 4-(6-Methoxy-3-nitro-pyridin-2-yl)-morpholine synthétisé selon le mode opératoire si dessous, 50ml d'éthanol, 20ml de cyclohexène et 2 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique, le précipité formé est essoré et séché sous vide jusqu'à poids constant.

On obtient 6.21g de poudre jaune Rdt=98.1%

Analyse en spectrométrie de masse, en spectroscopie de résonance magnétique : conformes.

| Analyse CHCINO : | | | | | |
|---|---|---|---|---|---|
| Trouvée : | C42.49 | H 6.40 | N 14.17 | O 15.70 | Cl 21.82 |
| Calculée : | C42.57 | H 6.07 | N 14.89 | O 11.34 | CI 25.13 |

### Exemple n°4 : N-[1-(3-amino-6-méthoxy-pyridin-2-yl)-pyrrolidin-3-yl]-acétamide

### A) Synthèse du : N-[1-(6-méthoxy-3-nitro-pyridin-2-yl)-pyrrolidin-3-yl]-acétamide

Dans un ballon tout équipé, on charge 2g (0.01 mole) de produit 2-chloro-3-nitro-6-méthoxypyridine, 30ml de dioxane, 5ml d'eau et 2.56 g (0.2mol)de 3acetamidopyrrolidine.

Le mélange est porté au reflux pendant deux heures sous agitation puis le mélange est versé sur un mélange de glace eau sous agitation. Le précipité formé est essoré et séché sous vide jusqu'à poids constant.

On obtient 2.51g de poudre jaune.

Analyse en spectrométrie de masse, en spectroscopie de résonance magnétique : conformes.

### B) Synthèse du N-[1-(3-Amino-6-méthoxy-pyridin-2-yl)-pyrrolidin-3-yl]-acétamide

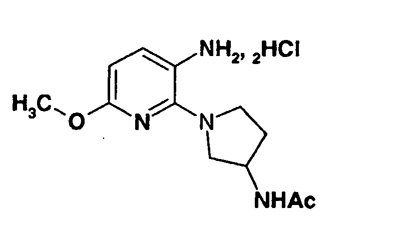

Dans un ballon tout équipé, on charge 2.35g (0.0084 mole) de produit -[1-(6-méthoxy-3-nitro-pyridin-2-yl)-pyrrolidin-3-yl] acétamide synthétisé selon le mode opératoire si dessous, 15ml d'éthanol, 7ml de cyclohexene et 0.7 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 2.05g de poudre marron.

Analyse en spectrométrie de masse, en spectroscopie de résonance magnétique: conformes

| Analyse CHCINO : | | | | | |
|---|---|---|---|---|---|
| Trouvée : | C 50.26 | H 6.68 | N 19.54 | O 11.16 | CI 12.36 |
| Calculée : | C 50.16 | H 6.83 | N 19.42 | O 11.30 | CI 12.42 |

### Exemple n°5 : 6'-méthoxy-3,4,5,6-tetrahydro-2H-[1,2']bipyridinyl-3'-ylamine:

### A) Synthèse du 6'-méthoxy-3'-nitro-3,4,5,6-tetrahydro-2H-[1,2']bipyridinyl:

Dans un ballon tout équipé, on charge .1.9g (0.01 mole)de produit 2-chloro-3-nitro-6-méthoxypyridine, 30ml de dioxane, 5ml d'eau et1.98ml (0.02mol) de pipéridine.

Le mélange est porté au reflux pendant deux heures sous agitation puis le mélange est versé sur un mélange de glace eau sous agitation. Le précipité formé est essoré et séché sous vide jusqu'à poids constant.

On obtient 2.14g de poudre jaune.

Analyse en spectrométrie de masse, en spectroscopie de résonance magnétique : conformes

### B) Synthèse du 6'-méthoxy-3,4,5,6-tetrahydro-2H-[1,2']bipyridinyl-3'-ylamine:

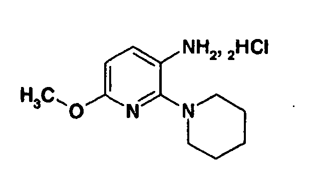

Dans un ballon tout équipé, on charge 2g (0.0084 mole) de produit 6'-méthoxy-3'-nitro-3,4,5,6-tetrahydro-2H-[1,2']bipyridinyl synthétisé selon le mode opératoire si dessous, 50ml d'éthanol, 5ml de cyclohexène et 0.5 g de palladium sur charbon.
Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 1.57g de poudre Rdt = 77%.

Analyse en spectrométrie de masse, en spectroscopie de résonance magnétique: conformes.

| Analyse CHClNO : | | | | | |
|---|---|---|---|---|---|
| Trouvée : | C 47.19 | H 6.81 | N 14.81 | O 6.45 | CI 25.44 |
| Calculée : | C 47.15 | H 6.83 | N 15 | O 5.71 | CI 25.31 |

### Exemple n°6 : 2-(3'-amino-6'-méthoxy-3,4,5,6-tetrahydro-2H-1 [1,2']bipyridinyl-2-yl)-éthanol:

### A) Synthèse du 2-(6'-méthoxy-3'-nitro-3,4,5,6-tetrahydro-2H-I [1,2']bipyridinyl-2-yl)-éthanol:

Dans un ballon tout équipé, on charge 5g (0.0265 mole) de produit 2-chloro-3-nitro-6-méthoxypyridine, 50ml d'éthanol, 6.78ml (0.053mol) de pyrrolidine.

Le mélange est porté au reflux pendant deux heures sous agitation puis le mélange est versé sur un mélange de glace eau sous agitation. Le précipité formé est essoré et séché sous vide jusqu'à poids constant.

On obtient 7.01g de poudre jaune Rdt = 94.1%.

Analyse en spectrométrie de masse, en spectroscopie de résonance magnétique : conformes

### B) Synthèse du 2-(3'-amino-6'-méthoxy-3,4,5,6-tetrahydro-2H-1[1,2']bipyridinyl-2-yl)-éthanol:

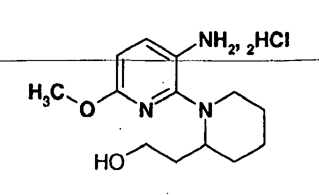

Dans un ballon tout équipé, on charge 6.5g (0.023 mole)de produit 2-(6'-Methoxy-3'-nitro-3,4,5,6-tetrahydro-2H-[1,2']bipyridinyl-2-yl)-éthanol synthétisé selon le mode opératoire si dessous, 50ml d'éthanol, 20ml de cyclohexene et 1.5 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 6.46g de poudre Rdt = 97.9%.

Analyse en spectrométrie de masse, en spectroscopie de résonance magnétique: conformes.

| Analyse CHClNO : | | | | | |
|---|---|---|---|---|---|
| Trouvée : | C 48.02 | H 7.23 | N 12.25 | 012.41 | Cl 19.36 |
| Calculée: | C 48.16 | H 7.15 | N 12.96 | O 9.87 | CI 21.87 |

### Exemple n°7 : 6-Methoxy-2-(4-methyl-piperazin-1-yl)-pyridin-3-ylamine:

### A) Synthèse du 1-(6-méthoxy-3-nitro-pyridin-2-yl)-4-méthyl-piperazine :

Dans un ballon tout équipé, on charge 1.9g (0.01 mole) de produit 2-chloro-3-nitro-6-méthoxypyridine, 30ml de dioxane, 5ml d'eau et 2.21ml (0.02mol) de 1-méthylpyperidine.

Le mélange est porté au reflux pendant deux heures sous agitation puis le mélange est versé sur un mélange de glace eau sous agitation. Le précipité formé est essoré et séché sous vide jusqu'à poids constant.

On obtient 1.022g de poudre jaune Rdt = 41 %.

Analyse en spectrométrie de masse, en spectroscopie de résonance magnétique : conformes.

### B) Synthèse du 6-méthoxy-2-(4-méthyl-piperazin-1-yl)-pyridin-3-ylamine

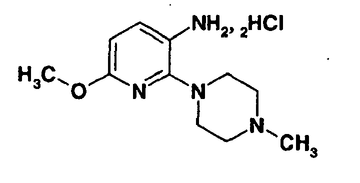

Dans un ballon tout équipé, on charge 1.30g (0.0052 mole)de produit 1-(6-Methoxy-3-nitro-pyridin-2-yl)-4-methyl-piperazine synthétisé selon le mode opératoire si dessous, 25ml d'éthanol, 5ml de cyclohexene et 0.6 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 1.024g de poudre Rdt = 77%.

Analyse en spectrométrie de masse, en spectroscopie de résonance magnétique : conformes.

| Analyse CHNOCl | | | | | |
|---|---|---|---|---|---|
| Trouvée: | C 43.12 | H 7.02 | N | O 9.47 | Cl 22.77 |
| Calculée : | C 44.76 | H 6.83 | N 18.98 | O 5.42 | Cl 24.02 |

### Exemple n°8 : 6'-méthoxy-2-méthyl-3,4,5,6-tetrahydro-2H-[1,2']bipyridinyl-l3'-ylamine:

### A) Synthèse du 6'-Methoxy-2-methyl-3'-nitro-3,4,5,6-tetrahydro-2H-[1,2']bipyridinyl:

Dans un ballon tout équipé, on charge 5g (0.027 mole)de produit 2-chloro-3-nitro-6-méthoxypyridine, 50ml d'éthanol et 6.26ml (0.053mol)de2-methyl pipéridine.

Le mélange est porté au reflux pendant deux heures sous agitation puis le mélange est versé sur un mélange de glace eau sous agitation. Le précipité formé est essoré et séché sous vide jusqu'à poids constant.

On obtient 6.48g de poudre jaune Rdt=100%.

Analyse en spectrométrie de masse, en spectroscopie de résonance magnétique : conformes.

### B) Synthèse du 6'-méthoxy-2-méthyl-3,4,5,6-tetrahydro-2H-[1,2']bipyridinyl-13'-ylamine

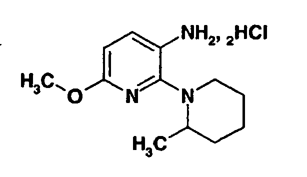

Dans un ballon tout équipé, on charge 5g (0.02 mole)de produit 6'-méthoxy-2-méthyl-3'-nitro-3,4,5,6-tetrahydro-2H-[1,2']bipyridinyl synthétisé selon le mode opératoire si dessous, 50ml d'ethanol, 20ml de cyclohexène et 1.5 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 5.1g de poudre Rdt=100%.

Analyse en spectrométrie de masse, en spectroscopie de résonance magnétique : conformes

| Analyse CHClNO : | | | | | |
|---|---|---|---|---|---|
| Trouvée : | C 48.79 | H7.16 | N 14.04 | O 7.04 | Cl 22.01 |
| Calculée : | C 48.99 | H7.19 | N 14.28 | O 5.44 | Cl 24.1 |

### Exemple n°9: 6'-méthoxy-3-méthyl-3,4,5,6-tetrahydro-2H-[1,2']bipyridinyl-3'-ylamine

### A) Synthèse du 6-méthoxy-3-méthyl-3'-nitro-3,4,5,6-tetrahydro-2H-[1,2]bipyridinyl

Dans un ballon tout équipé, on charge 5g (0.027 mole)de produit 2-chloro-3-nitro-6-méthoxypyridine, 50ml d'éthanol et 6.26ml (0.053mol)de2-methyl pipéridine.

Le mélange est porté au reflux pendant deux heures sous agitation puis le mélange est versé sur un mélange de glace eau sous agitation. Le précipité formé est essoré et séché sous vide jusqu'à poids constant.

On obtient 6.41g de poudre jaune Rdt= 96,4%.

Analyse en spectrométrie de masse, en spectroscopie de résonance magnétique : conformes.

### B) Synthèse du 6'-méthoxy-3-méthyl-3,4,5,6-tetrahydro-2H-[1,2']bipyridinyl-3'-ylamine

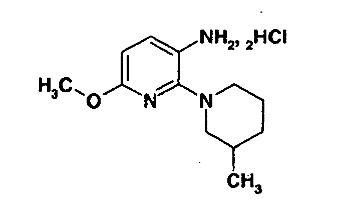

Dans un ballon tout équipé, on charge 6.25g (0.025 mole) de produit 6'-Methoxy-3-methyl-3'-nitro-3,4,5,6-tetrahydro-2H-[1,2']bipyridinyl synthétisé selon le mode opératoire si dessous, 50ml d'ethanol, 20ml de cyclohexène et 2.0 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 5.51g de poudre Rdt = 86%.

Analyse en spectrométrie de masse, en spectroscopie de résonance magnétique : conformes.

| Analyse CHClNO : | | | | | |
|---|---|---|---|---|---|
| Trouvée : | C 50.31 | H 7.22 | N 13.99 | O 7.33 | CI 20.16 |
| Calculée : | C 48.99 | H 7.19 | N 14.28 | O 5.44 | Cl 24.1 |

### Exemple n°10: 2-(3'-amino-6'-méthoxy-3,4,5,6-tetrahydro-2H-[1,2']bipyridinyl-2-yl)-éthanol

### A) Synthèse du 2-(6'-Methoxy-3'-nitro-3,4,5,6-tetrahydro-2H-[1,2']bipyridinyl-2-yl)-éthanol :

Dans un ballon tout équipé, on charge 5g (0.027 mole)de produit 2-chloro-3-nitro-6-méthoxypyridine, 50ml d'éthanol et 6.78ml (0.053mol)de2-ethanol pipéridine.

Le mélange est porté au reflux pendant deux heures sous agitation puis le mélange est versé sur un mélange de glace eau sous agitation. Le précipité formé est essoré et séché sous vide jusqu'à poids constant.

On obtient 7.01 g de poudre jaune Rdt = 94.1 %.

Analyse en spectrométrie de masse, en spectroscopie de résonance magnétique : conformes.

### B) Synthèse du 2-(3'-amino-6'-méthoxy-3,4,5,6-tetrahydro-2H-[1,2']bipyridinyl-2-yl)-éthanol

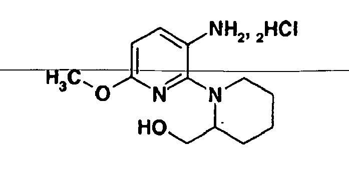

Dans un ballon tout équipé, on charge 6.5g (0.023 mole)de produit 2-(6'-Methoxy-3'-nitro-3,4,5,6-tetrahydro-2H [1,2']bipyridinyl-2-yl)-éthanol synthétisé selon le mode opératoire si dessous, 50ml d'éthanol, 20ml de cyclohexene et 1.5 g de palladium sur charbon.

Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant.

On obtient 6.46g de poudre Rdt = 97.9%.

Analyse en spectrométrie de masse, en spectroscopie de résonance magnétique : conformes.

| Analyse CHClNO : | | | | | |
|---|---|---|---|---|---|
| Trouvée : | C 48.02 | H 7.23 | N12.25 | O 12.41 | CI 19.36 |
| Calculée : | C 48.16 | H 7.15 | N12.96 | O 9.87 | Cl 21.87 |

### EXEMPLES DE TEINTURE

On a préparé les compositions tinctoriales suivantes

| Exemples | 1 | 2 |
|---|---|---|
| Paraphénylènediamine | 5.10⁻³ mole | - |
| N,N-Bis-(2-hydroxyethyl)-p-phenylenediamine) | - | 5.10⁻³ mole |
| 6-méthoxy-2-pyrrolidin-1-yl-pyridin-3-ylamine | 5.10⁻³ mole | 5.10⁻³ mole |
| Support de teinture | (1) | (1) |
| Eau déminéralisée q.s.p. | 100 g | 100 g |

### Support de teinture (1) pH 7

| | |
|---|---|
| Alcool éthylique à 96° | 20 g |
| Métabisulfite de sodium en solution aqueuse à 35% | 0,2275gM.A |
| sel pentasodique de l'acide diethylene triamino pentacétique | 0,48 g M.A |
| Alkyl en C₈-C₁₅polyglucoside vendu en solution à 60% sous la dénomination ORAMIXCG110 par la société SEPPIC | 3,6 g M.A |
| Alcool benzylique | 2,0 g |
| Polyethylène glycol à 8 moles d'OE | 3,0 g |
| K₂HPO₄ | 20,9 g |
| KH₂PO₄ | 10,88g |

Au moment de l'emploi, chaque composition est mélangée avec un poids égal d'eau oxygénée à 20 volumes (6% en poids). On obtient un pH final de 7, ou 9,5 selon le support utilisé.

Chaque mélange obtenu est appliqué sur des mèches de cheveux gris à 90 % de blancs. Après 30 min de pose, les mèches sont lavées avec un shampooing standard, rinçées puis séchées.

Chaque mèche est évaluée avant et après la teinture dans le système L*a*b*, au moyen d'un spectrophotomètre CM 2002 MINOLTA ®, (Illuminant D65).

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

Les résultats de teinture suivants ont été obtenus

| | **Cheveux naturels** | | |
|---|---|---|---|
| | **L*** | **a*** | **b*** |
| **Exemple 1** | 22.24 | 0.1 | -3.4 |
| **Exemple 2** | 29.75 | -6.68 | -9.3 |

## Revendications

1. Composition tinctoriale comprenant, dans un milieu de teinture approprié :
- au moins une base d'oxydation, et
- au moins un coupleur 6-alcoxy-2,3-diaminopyridine de formule (I) ou l'un de ses sels d'addition correspondants : 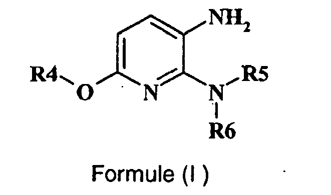
dans laquelle :
- R₄ représente un radical alkyle en C₁-C₄ éventuellement substitué par un ou plusieurs radicaux hydroxyle, alcoxy en C₁-C₂, NR₇R₈ où R₇ et R₈ sont choisis parmi l'hydrogène, les radicaux alkyle en C₁-C₄, (poly)hydroxyalkyle en C₂-C₆, (poly)aminoalkyle en C₂-C₆, amino-hydroxyalkyle en C₂-C₆ ;
- R₅ et R₆ forment avec l'atome d'azote avec lequel ils sont attachés un cycle comportant 5 à 8 chaînons dont un ou plusieurs atomes de carbone du cycle carboné peuvent être remplacés par un atome d'oxygène, d'azote éventuellement substitué, de soufre ou par un groupement SO₂, et dont les atomes de carbone peuvent être, indépendamment les uns des autres, substitués ; ledit cycle ne comportant pas de liaison peroxyde, ni de radicaux diazo ou nitroso.

2. Composition selon la revendication 1 , dans laquelle R₅ et R₆ d'autre part, forment avec l'atome d'azote auquel ils sont rattachés un hétérocycle de 5 à 8 chaînons choisi parmi les hétérocycles pyrrolidine, pipéridine, homopipéridine, pipérazine, homopipérazine, diazépane, lesdits cycles pouvant être substitués par un ou plusieurs radicaux choisis parmi les radicaux :
(i) alkyle en C₁-C₄ éventuellement substitué par un ou plusieurs radicaux choisis parmi les radicaux hydroxy, amino, (di)alkylamino en C₁-C₂, carboxy ;
(ii) hydroxy,
(iii) amino,
(iv) (di)alkylamino en C₁-C₂ ,
(v) carboxy,
(vi) carboxamido,
(vii) sulfonamido.

3. Composition selon la revendication 1, dans laquelle R₅ et R₆ forment un hétérocycle choisi parmi la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la 3-acétamidopyrrolidine, la 3-(méthylsulfonylamino)-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, le diazépane, le N-méthyl diazépane, le N-(2-hydroxyéthyl)- diazépane.

4. Composition selon la revendication 3, dans laquelle R₅ et R₆ forment un hétérocycle choisi parmi la pyrrolidine, la 3-hydroxypyrrolidine, la 3-aminopyrrolidine, la 3-acétamidopyrrolidine, la 3-(méthylsulfonylamino)-pyrrolidine, la proline, la 3-hydroxyproline, la pipéridine, l'hydroxypipéridine, l'homopipéridine, le diazépane, N-méthyl homopipérazine, la N β-hydroxyéthylhomopipérazine et leurs sels d'addition.

5. Composition selon la revendication 1, dans laquelle le composé de formule (I) est choisi parmi :
- la 6-méthoxy-2-pyrrolidin-1-yl-pyridin-3-ylamine ;
- la 2-(2,5-diméthyl-pyrrolidin-1-yl)-6-méthoxy-pyridin-3- ylamine ;
- la 6-méthoxy-2-morpholin-4-yl-pyridin-3-ylamine ;
- le N-[1-(3-amino-6-méthoxy-pyridin-2-yl)-pyrrolidin-3-yl]-acétamide ;
- la 6'-méthoxy-3,4,5,6-tetrahydro-2H-[1,2']bipyridinyl-3'-ylamine ;
- la 2-(3'-Amino-6'-methoxy-3,4,5,6-tetrahydro-2H-I [1,2']bipyridinyl-2-yl)-éthanol ;
- la 6-méthoxy-2-(4-méthyl-pipérazin-1-yl)-pyridin-3-ylamine ;
- la 6 '-méthoxy-2-méthyl-3, 4,5,6-tetrahydro-2H-[1,2']bipyridinyl-I3'-ylamine ;
- la 6'-méthoxy-3-methyl-3,4,5,6-tetrahydro-2H-[1,2']bipyridinyl-3'-ylamine;
- la 2-(3'-Amino-6'-méthoxy-3,4,5,6-tetrahydro-2H-[1,2']-bipyridinyl-2-yl)-éthanol ainsi que leurs sels d'addition.

6. Composition selon l'une quelconque des revendications 1 à 5, où la ou les bases d'oxydation sont choisies parmi les paraphénylènediamines, les bis-phénylalkylènediamines, les para-aminophénols, les ortho-aminophénols, les bases hétérocycliques et leurs sels d'addition.

7. Composition selon l'une quelconque des revendications 1 à 6, dans laquelle la ou les bases d'oxydation sont chacune présentes en quantité comprise 0,001 et 10 % en poids du poids total de la composition tinctoriale.

8. Composition selon l'une quelconque des revendications 1 à 7, comprenant un ou plusieurs coupleurs additionnels choisis parmi les métaphénylènediamines, les métaaminophénols, les métadiphénols, les coupleurs naphtaléniques et les coupleurs hétérocycliques et leurs sels d'addition.

9. Composition selon l'une quelconque des revendications 1 à 8, dans laquelle le ou les coupleurs sont présents chacun en quantité comprise entre 0,001 et 10 % en poids du poids total de la composition tinctoriale.

10. Procédé de teinture d'oxydation des fibres kératiniques **caractérisé en ce qu'**on applique sur les fibres une composition telle que définie à l'une quelconque des revendications 1 à 9, et qu'on révèle la couleur à l'aide d'un agent oxydant.

11. Procédé selon la revendication 10, dans lequel l'agent oxydant est choisi parmi le peroxyde d'hydrogène, le peroxyde d'urée, les bromates de métaux alcalins, les persels, les peracides et les enzymes oxydases.

12. Procédé selon l'une des revendications 9 ou 10 dans lequel l'agent oxydant est mélangé au moment de l'emploi à la composition telle que définie selon l'une quelconque des revendications 1 à 9.

13. Procédé selon l'une quelconque des revendications 10 à 12, dans lequel l'agent oxydant est appliqué sur les fibres simultanément ou séquentiellement à la composition telle que définie selon l'une quelconque des revendications 1 à 9 sous forme d'une composition oxydante.

14. Dispositif à plusieurs compartiments ou "kit" de teinture à plusieurs compartiments, dans lequel un premier compartiment tinctoriale contient une composition telle que définie à l'une quelconque des revendications 1 à 9 et un deuxième compartiment contient une composition oxydante.

15. Utilisation de la composition telle que définie selon l'une quelconque des revendications 1 à 9 pour la teinture des fibres kératiniques.

16. Composé 6-alcoxy-2,3-diaminepyridine de formule (I) suivante : 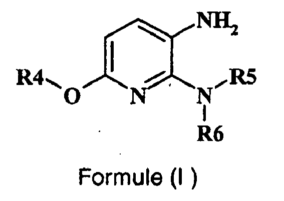 dans laquelle :
- R₄ représente un radical alkyle en C₁-C₄ éventuellement substitué par un ou plusieurs radicaux hydroxyle, alcoxy en C₁-C₂ , NR₇R₈ où R₇ et R₈ sont choisis dans un groupe constitué par un atome d'hydrogène, un alkyle en C₁-C₄, un (poly)hydroxyalkyle en C₂-C₆, un (poly)aminoalkyle en C₂-C₆, un amino-hydroxyalkyle en C₂-C₆ ;
- R₅ et R₆ forment avec l'atome d'azote avec lequel ils sont attachés un cycle comportant 5 à 8 chaînons dont un ou plusieurs atomes de carbone du cycle carboné peuvent être remplacés par un atome d'oxygène, d'azote éventuellement substitué ou de soufre ou par un groupement SO₂, et dont les atomes de carbone peuvent être, indépendamment les uns des autres, substitués ; ledit cycle ne comportant pas de liaison peroxyde, ni de radicaux diazo ou nitroso ainsi que ses sels d'addition à l'exception des composés 3-amino 6-méthoxy 2-(4-méthyl 2-propylimidazol-1-yl)-pyridine et 3-amino 6-méthoxy 2-pyrrolo-pyridine.

17. Composé selon la revendication 16 , dans laquelle R₅ et R₆ forment avec l'atome d'azote auquel ils sont rattachés un hétérocycle de 5 à 8 chaînons choisi parmi les hétérocycles pyrrolidine, pipéridine, homopipéridine, pipérazine, homopipérazine, diazépane.

18. Composés selon la revendication 17 dans laquelle lesdits hétérocycles peuvent être substitués par un ou plusieurs radicaux choisis parmi :
(viii) alkyle en C₁-C₄ éventuellement substitué par un ou plusieurs radicaux choisis dans le groupe constitué par un hydroxy, amino, (di)alkylamino en C₁-C₂, carboxy ;
(ix) hydroxy,
(x) amino,
(xi) (di)alkylamino en C₁-C₂ ,
(xii) carboxy,
(xiii) carboxamido
(xiv) ou sulfonamido.

19. Composé selon la revendication 16, dans laquelle R₅ et R₆ forment un hétérocycle choisi parmi la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la 3-acétamidopyrrolidine, la 3-(méthylsulfonylamino)-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, le diazépane, le N-méthyl diazépane, le N-(2-hydroxyéthyl)- diazépane.

20. Composé selon la revendication 19, dans laquelle R₅ et R₆ forment un hétérocycle choisi parmi la pyrrolidine, la 3-hydroxypyrrolidine, la 3-aminopyrrolidine, la 3-acétamidopyrrolidine, la 3-(méthylsulfonylamino)-pyrrolidine, la proline, la 3-hydroxyproline, la pipéridine, les hydroxypipéridines, l'homopipéridine, le diazépane, N-méthyl diazépane, la N β-hydroxyéthyldiazépane et leurs sels d'addition.

21. Composé selon la revendication 16, choisi parmi:
- la 6-méthoxy-2-pyrrolidin-1-yl-pyridin-3-ylamine ;
- la 2-(2,5-diméthyl-pyrrolidin-1-yl)-6-méthoxy-pyridin-3- ylamine ;
- la 6-méthoxy-2-morpholin-4-yl-pyridin-3-ylamine ;
- le N-[1-(3-amino-6-méthoxy-pyridin-2-yl)-pyrrolidin-3-yl]-acétamide ;
- la 6'-méthoxy-3,4,5,6-tetrahydro-2H-[1,2']bipyridinyl-3'-ylamine ;
- la 2-(3'-Amino-6'-methoxy-3,4,5,6-tetrahydro-2H-I [1,2']bipyridinyl-2-yl)-éthanol ;
- la 6-méthoxy-2-(4-méthyl-pipérazin-1-yl)-pyridin-3-ylamine ;
- la 6'-méthoxy-2-méthyl-3,4,5,6-tetrahydro-2H-[1,2']bipyridinyl-I3'-ylamine ;
- la 6'-méthoxy-3-methyl-3,4,5,6-tetrahydro-2H-[1,2']bipyridinyl-3'-ylamine;
- la 2-(3'-Amino-6'-méthoxy-3,4,5,6-tetrahydro-2H-[1,2']-bipyridinyl-2-yl)-éthanol ainsi que leurs sels d'addition.

## Claims

1. Dye composition comprising, in a medium that is suitable for dyeing:
- at least one oxidation base, and
- at least one 6-alkoxy-2,3-diaminopyridine coupler of formula (I) or one of the corresponding addition salts thereof: 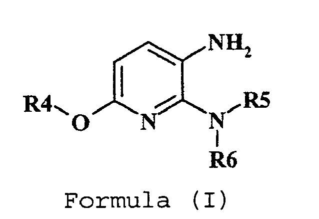
in which:
- R₄ represents a C₁-C₄ alkyl radical optionally substituted with one or more radicals chosen from hydroxyl, C₁-C₂ alkoxy and NR₇R₈ radicals, in which R₇ and R₈ are chosen from hydrogen and C₁-C₄ alkyl, C₂-C₆ (poly)hydroxyalkyl, C₂-C₆ (poly) aminoalkyl and C₂-C₆ aminohydroxyalkyl radicals;
- R₅ and R₆ form, with the nitrogen atom to which they are attached, a 5- to 8-membered ring, of which one or more carbon atoms of the carbon ring may be replaced with an oxygen atom, an optionally substituted nitrogen atom, a sulphur atom, or an SO₂ group, and the carbon atoms of which may be substituted, independently of each other; the said ring not comprising a peroxide bond, or diazo or nitroso radicals.

2. Composition according to Claim 1, in which R₅ and R₆ moreover form, with the nitrogen atom to which they are attached, a 5- to 8-membered heterocycle chosen from pyrrolidine, piperidine, homopiperidine, piperazine, homopiperazine and diazepane heterocycles, the said rings possibly being substituted with one or more radicals chosen from the following radicals:
(i) C₁-C₄ alkyl optionally substituted with one or more radicals chosen from hydroxyl, amino, C₁-C₂ (di)alkylamino and carboxyl radicals;
(ii) hydroxyl;
(iii) amino;
(iv) C₁-C₂ (di)alkylamino;
(v) carboxyl;
(vi) carboxamido;
(vii) sulphonamido.

3. Composition according to Claim 1, in which R₅ and R₆ form a heterocycle chosen from 2,5-dimethylpyrrolidine, proline, 3-hydroxyproline, 4-hydroxyproline, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 4-amino-3-hydroxypyrrolidine, 3-hydroxy-4-(2-hydroxyethyl)aminopyrrolidine, 3-acetamidopyrrolidine, 3-(methylsulphonylamino)pyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, diazepane, N-methyldiazepane and N-(2-hydroxyethyl)diazepane.

4. Composition according to Claim 3, in which R₅ and R₆ form a heterocycle chosen from pyrrolidine, 3-hydroxypyrrolidine, 3-aminopyrrolidine, 3-acetamidopyrrolidine, 3-(methylsulphonylamino)-pyrrolidine, proline, 3-hydroxyproline, piperidine, hydroxypiperidine, homopiperidine, diazepane, N-methylhomopiperazine and N-β-hydroxyethylhomopiperazine, and the addition salts thereof.

5. Composition according to Claim 1, in which the compound of formula (I) is chosen from:
- 6-methoxy-2-pyrrolidin-1-ylpyrid-3-ylamine;
- 2-(2,5-dimethylpyrrolidin-1-yl)-6-methoxypyrid-3-ylamine;
- 6-methoxy-2-morpholin-4-ylpyrid-3-ylamine;
- N-[1-(3-amino-6-methoxypyrid-2-yl)pyrrolidin-3-yl]acetamide;
- 6'-methoxy-3,4,5,6-tetrahydro-2H-[1,2']bipyridyl-3'-ylamine;
- 2-(3'-amino-6'-methoxy-3,4,5,6-tetrahydro-2H-[1,2']bipyridyl-2-yl)ethanol;
- 6-methoxy-2-(4-methylpiperazin-1-yl)pyrid-3-ylamine;
- 6'-methoxy-2-methyl-3,4,5,6-tetrahydro-2H-[1,2']bipyridyl-3'-ylamine;
- 6'-methoxy-3-methyl-3,4,5,6-tetrahydro-2H-[1,2']bipyridyl-3'-ylamine;
- 2-(3'-amino-6'-methoxy-3,4,5,6-tetrahydro-2H-[1,2']bipyridyl-2-yl)ethanol, and also the addition salts thereof.

6. Composition according to any one of Claims 1 to 5, in which the oxidation base(s) is (are) chosen from para-phenylenediamines, bis(phenyl)alkylenediamines, para-aminophenols, ortho-aminophenols and heterocyclic bases, and the addition salts thereof.

7. Composition according to any one of Claims 1 to 6, in which the oxidation base(s) is (are) each present in an amount of between 0.001% and 10% by weight relative to the total weight of the dye composition.

8. Composition according to any one of Claims 1 to 7, comprising one or more additional couplers chosen from meta-phenylenediamines, meta-aminophenols, meta-diphenols, naphthalenic couplers and heterocyclic couplers, and the addition salts thereof.

9. Composition according to any one of Claims 1 to 8, in which the coupler(s) is (are) each present in an amount of between 0.001% and 10% by weight relative to the total weight of the dye composition.

10. Process for the oxidation dyeing of keratin fibres, **characterized in that** a composition as defined in any one of Claims 1 to 9 is applied to the fibres and the colour is developed using an oxidizing agent.

11. Process according to Claim 10, in which the oxidizing agent is chosen from hydrogen peroxide, urea peroxide, alkali metal bromates, persalts, peracids and oxidase enzymes.

12. Process according to either of Claims 9 and 10, in which the oxidizing agent is mixed at the time of use with the composition as defined according to any one of Claims 1 to 9.

13. Process according to any one of Claims 10 to 12, in which the oxidizing agent is applied to the fibres simultaneously with or sequentially to the composition as defined according to any one of Claims 1 to 9, in the form of an oxidizing composition.

14. Multi-compartment device or multi-compartment dyeing kit, in which a first dyeing compartment contains a composition as defined in any one of Claims 1 to 9 and a second compartment contains an oxidizing composition.

15. Use of the composition as defined according to any one of Claims 1 to 9, for dyeing keratin fibres.

16. 6-Alkoxy-2,3-diaminopyridine compound of formula (I) below: 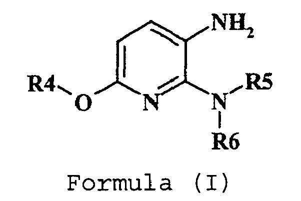 in which:
- R₄ represents a C₁-C₄ alkyl radical optionally substituted with one or more radicals chosen from hydroxyl, C₁-C₂ alkoxy and NR₇R₈ radicals, in which R₇ and R₈ are chosen from a group comprising a hydrogen atom, a C₁-C₄ alkyl, a C₂-C₆ (poly) hydroxyalkyl, a C₂-C₆ (poly) aminoalkyl and a C₂-C₆ aminohydroxyalkyl ;
- R₅ and R₆ form, with the nitrogen atom to which they are attached, a 5- to 8-membered ring, of which one or more carbon atoms of the carbon ring may be replaced with an oxygen atom, an optionally substituted nitrogen atom, a sulphur atom, or an SO₂ group, and the carbon atoms of which may be substituted, independently of each other; the said ring not comprising a peroxide bond, or diazo or nitroso radicals or the addition salts thereof with the exception of the compounds 3-amino-6-methoxy-2-(4-methyl-2-propylimidazol-1-yl)pyridine and 3-amino-6-methoxy-2-pyrrolopyridine.

17. Compound according to Claim 16, in which R₅ and R₆ form, with the nitrogen atom to which they are attached, a 5- to 8-membered heterocycle chosen from pyrrolidine, piperidine, homopiperidine, piperazine, homopiperazine and diazepane heterocycles.

18. Compound according to Claim 17, in which the said heterocycles may be substituted with one or more radicals chosen from the following radicals:
(viii) C₁-C₄ alkyl optionally substituted with one or more radicals chosen from hydroxyl, amino, C₁-C₂ (di)alkylamino and carboxyl radicals;
(ix) hydroxyl;
(x) amino;
(xi) C₁-C₂ (di)alkylamino;
(xii) carboxyl;
(xiii) carboxamido;
(xiv) sulphonamido.

19. Compound according to Claim 16, in which R₅ and R₆ form a heterocycle chosen from 2,5-dimethylpyrrolidine, proline, 3-hydroxyproline, 4-hydroxyproline, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxy-pyrrolidine, 3,4-dihydroxypyrrolidine, 3-amino-pyrrolidine, 3-methylaminopyrrolidine, 4-amino-3-hydroxypyrrolidine, 3-hydroxy-4-(2-hydroxyethyl)-aminopyrrolidine, 3-acetamidopyrrolidine, 3-(methylsulphonylamino)pyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, diazepane, N-methyldiazepane and N-(2-hydroxyethyl)diazepane.

20. Compound according to Claim 19, in which R₅ and R₆ form a heterocycle chosen from pyrrolidine, 3-hydroxypyrrolidine, 3-aminopyrrolidine, 3-acetamidopyrrolidine, 3-(methylsulphonylamino)-pyrrolidine, proline, 3-hydroxyproline, piperidine, hydroxypiperidines, homopiperidine, diazepane, N-methyldiazepane and N-β-hydroxyethyldiazepane, and the addition salts thereof.

21. Compound according to Claim 16, chosen from:
- 6-methoxy-2-pyrrolidin-1-ylpyrid-3-ylamine;
- 2-(2,5-dimethylpyrrolidin-1-yl)-6-methoxypyrid-3-ylamine;
- 6-methoxy-2-morpholin-4-ylpyrid-3-ylamine;
- N-[1-(3-amino-6-methoxypyrid-2-yl)pyrrolidin-3-yl]acetamide;
- 6'-methoxy-3,4,5,6-tetrahydro-2H-[1,2']bipyridyl-3'-ylamine;
- 2-(3'-amino-6'-methoxy-3,4,5,6-tetrahydro-2H-[1,2']bipyridyl-2-yl)ethanol;
- 6-methoxy-2-(4-methylpiperazin-1-yl)pyrid-3-ylamine;
- 6'-methoxy-2-methyl-3,4,5,6-tetrahydro-2H-[1,2']bipyridyl-3'-ylamine;
- 6'-methoxy-3-methyl-3,4,5,6-tetrahydro-2H-[1,2']bipyridyl-3'-ylamine;
- 2-(3'-amino-6'-methoxy-3,4,5,6-tetrahydro-2H-[1,2']bipyridyl-2-yl)ethanol, and also the addition salts thereof.

## Patentansprüche

1. Farbmittelzusammensetzung, die in einem geeigneten Farbmittelmedium enthält:
- mindestens eine Oxidationsbase, und
- mindestens einen 6-Alkoxy-2,3-diaminopyridinkuppler der Formel (I) oder eines seiner entsprechenden Additionssalze: 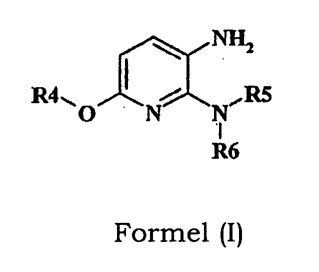
wobei in der Formel:
- R₄ eine C₁₋₄-Alkylgruppe bedeutet, die gegebenenfalls mit einer oder mehreren Gruppen Hydroxy, C₁₋₂-Alkoxy, NR₇R₈ substituiert ist, wobei R₇ und R₈ unter Wasserstoff, C₁₋₄-Alkyl, C₂₋₆-(Poly)hydroxyalkyl, C₂₋₆-(Poly)aminoalkyl, C₂₋₆-Aminohydroxyalkyl ausgewählt sind;
- R₅ und R₆ bilden mit dem Stickstoffatom, an das sie gebunden sind, einen 5- bis 8-gliedrigen Ring, bei dem ein oder mehrere Kohlenstoffatome des Kohlenstoffrings durch ein Sauerstoffatom, ein gegebenenfalls substituiertes Stickstoffatom, ein Schwefelatom oder eine SO₂-Gruppe ersetzt sein können und bei dem die Kohlenstoffatome unabhängig voneinander substituiert sein können; wobei der Ring keine Peroxidbindung und keine Diazogruppe oder Nitrosogruppe enthält.

2. Zusammensetzung nach Anspruch 1, wobei R₅ und R₆ mit dem Stickstoffatom an das sie gebunden sind, einen 5- bis 8-gliedrigen Heterocyclus bilden, der unter den Heterocyclen Pyrrolidin, Piperidin, Homopiperidin, Piperazin, Homopiperazin, Diazepan ausgewählt ist, wobei die Ringe mit einer oder mehreren Gruppen substituiert sein können, die unter den folgenden Gruppen ausgewählt sind:
(i) C₁₋₄-Alkyl, wobei sie gegebenenfalls mit einer oder mehreren Gruppen substituiert ist, die unter den Gruppen Hydroxy, Amino, C₁₋₂-(Di)alkylamino, Carboxy ausgewählt sind;
(ii) Hydroxy,
(iii) Amino,
(iv) C₁₋₂-(Di)alkylamino,
(v) Carboxy,
(vi) Carboxamido,
(vii) Sulfonamido.

3. Zusammensetzung nach Anspruch 1, wobei R₅ und R₆ einen Heterocyclus bilden, der unter 2,5-Dimethoxypyrrolidin, Prolin, 3-Hydroxyprolin, 4-Hydroxyprolin, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)-pyrrolidin, 2-Hydroxymethylpyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 4-Amino-3-hydroxypyrrolidin, 3-Hydroxy-4-(2-hydroxyethyl)aminopyrrolidin, 3-Acetamidopyrrolidin, 3-(Methylsulfonylamino)-pyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Diazepan, N-Methyldiazepan, N-(2-Hydroxyethyl)-diazepan ausgewählt ist.

4. Zusammensetzung nach Anspruch 3, wobei R₅ und R₆ einen Heterocyclus bilden, der unter Pyrrolidin, 3-Hydroxypyrrolidin, 3-Aminopyrrolidin, 3-Acetamidopyrrolidin, 3-(Methylsulfonylamino)-pyrrolidin, Prolin, 3-Hydroxyprolin, Piperidin, Hydroxypiperidin, Homopiperidin, Diazepan, N-Methylhomopiperazin, N-β-Hydroxyethylhomopiperazin und deren Additionssalzen ausgewählt ist.

5. Zusammensetzung nach Anspruch 1, wobei die Verbindung der Formel (I) ausgewählt ist unter:
- 6-Methoxy-2-pyrrolidin-1-yl-pyridin-3-ylamin;
- 2-(2,5-Dimethyl-pyrrolidin-1-yl)-6-methoxypyridin-3-ylamin;
- 6-Methoxy-2-morpholin-4-yl-pyridin-3-ylamin;
- N-[1-(3-Amino-6-methoxy-pyridin-2-yl)-pyrrolidin-3-yl]-acetamid;
- 6'-Methoxy-3,4,5,6-tetrahydro-2H-[1,2']bipyridinyl-3'-ylamin;
- 2-(3'-Amino-6'-methoxy-3,4,5,6-tetrahydro-2H-[1,2']bipyridinyl-2-yl)-ethanol;
- 6-Methoxy-2-(4-methyl-piperazin-1-yl)-pyridin-3-ylamin;
- 6'-Methoxy-2-methyl-3,4,5,6-tetrahydro-2H-[1,2']bipyridinyl-3'-ylamin;
- 6'-Methoxy-3-methyl-3,4,5,6-tetrahydro-2H-[1,2']bipyridinyl-3'-ylamin;
- 2-(3'-Amino-6'-methoxy-3,4,5,6-tetrahydro-2H-[1,2']bipyridinyl-2-yl)-ethanol
sowie deren Additionssalzen.

6. Zusammensetzung nach einem der Ansprüche 1 bis 5, wobei die Oxidationsbase oder die Oxidationsbasen unter den p-Phenylendiaminen, Bisphenylalkylendiaminen, p-Aminophenolen, o-Aminophenolen, heterocyclischen Basen und deren Additionssalzen ausgewählt sind.

7. Zusammensetzung nach einem der Ansprüche 1 bis 6, wobei die Oxidationsbase(n) jede in einer Menge von 0,001 bis 10 Gew.-% des Gesamtgewichts der Farbmittelzusammensetzung enthalten sind.

8. Zusammensetzung nach einem der Ansprüche 1 bis 7, die einen oder mehrere zusätzliche Kuppler enthält, die unter den *m-*Phenylendiaminen, *m*-Aminophenolen, *m*-Dihydroxybenzolen, Naphthalinkupplern und heterocyclischen Kupplern und deren Additionssalzen ausgewählt sind.

9. Zusammensetzung nach einem der Ansprüche 1 bis 8, wobei der oder die Kuppler jeweils in einer Menge von 0,001 bis 10 Gew.-% des Gesamtgewichts der Farbmittelzusammensetzung enthalten sind.

10. Verfahren zum oxidativen Färben von Keratinfasern, **dadurch gekennzeichnet, dass** auf die Fasern eine Zusammensetzung nach einem der Ansprüche 1 bis 9 aufgetragen wird und die Farbe mit einem Oxidationsmittel gebildet wird.

11. Verfahren nach Anspruch 10, wobei das Oxidationsmittel unter Wasserstoffperoxid, Harnstoffperoxid, Alkalimetallbromaten, Salzen von Persäuren, Persäuren und Oxidasen ausgewählt ist.

12. Verfahren nach einem der Ansprüche 9 oder 10, wobei das Oxidationsmittel bei der Anwendung mit der Zusammensetzung nach einem der Ansprüche 1 bis 9 vermischt wird.

13. Verfahren nach einem der Ansprüche 10 bis 12, wobei das Oxidationsmittel gleichzeitig mit der Zusammensetzung nach einem der Ansprüche 1 bis 9 in Form einer oxidierenden Zusammensetzung auf die Fasern aufgebracht wird oder getrennt davon anschließend.

14. Vorrichtung mit mehreren Abteilungen oder "Kit" zum Färben mit mehreren Abteilungen, wobei eine erste Farbmittelabteilung eine Zusammensetzung nach einem der Ansprüche 1 bis 9 und eine zweite Abteilung eine oxidierende Zusammensetzung enthält.

15. Verwendung der Zusammensetzung nach einem der Ansprüche 1 bis 9 zum Färben von Keratinfasern.

16. 6-Alkoxy-2,3-Diaminopyridin der folgenden Formel (I): 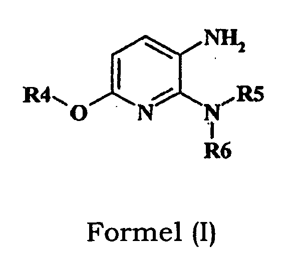 wobei in der Formel:
- R₄ eine C₁₋₄-Alkylgruppe bedeutet, die gegebenenfalls substituiert ist mit einer oder mehreren Gruppen Hydroxy, C₁₋₂-Alkoxy, NR₇R₈, wobei R₇ und R₈ unter Wasserstoff, C₁₋₄-Alkyl, C₂-₆-(Poly)hydroxyalkyl, C₂₋₆-(Poly)aminoalkyl, C₂₋₆-Aminohydroxyalkyl ausgewählt sind;
- R₅ und R₆ bilden mit dem Stickstoffatom, an das sie gebunden sind, einen 5- bis 8-gliedrigen Ring, bei dem ein oder mehrere Kohlenstoffatome des Kohlenstoffrings durch ein Sauerstoffatom, ein gegebenenfalls substituiertes Stickstoffatom, ein Schwefelatom oder eine SO₂-Gruppe ersetzt sein kann und bei dem die Kohlenstoffatome unabhängig voneinander substituiert sein können; wobei der Ring weder eine Peroxidbindung noch eine Diazo- oder Nitrosogruppe enthält; wobei 3-Amino-6-methoxy-(2-(4-methyl-2-propylimidazol-1-yl)-pyridin und 3-Amino-6-methoxy-2-pyrrolo-pyridin ausgenommen sind.

17. Verbindung nach Anspruch 16, wobei R₅ und R₆ mit dem Stickstoffatom an das sie gebunden sind, einen 5- bis 8-gliedrigen Heterocyclus bilden, der unter den Heterocyclen Pyrrolidin, Piperidin, Homopiperidin, Piperazin, Homopiperazin, Diazepan ausgewählt ist.

18. Verbindung nach Anspruch 17, wobei die Heterocyclen mit einer oder mehreren Gruppen substituiert sein können, die ausgewählt sind unter:
(i) C₁₋₄-Alkyl, die gegebenenfalls mit einer oder mehreren Gruppen substituiert ist, die unter den Gruppen Hydroxy, Amino, C₁₋₂-(Di)alkylamino, Carboxy ausgewählt sind;
(ii) Hydroxy,
(iii) Amino,
(iv) C₁₋₂-(Di)alkylamino,
(v) Carboxy,
(vi) Carboxamido,
(vii) Sulfonamido.

19. Verbindung nach Anspruch 16, wobei R₅ und R₆ einen Heterocyclus bilden, der unter 2,5-Dimethoxypyrrolidin, Prolin, 3-Hydroxyprolin, 4-Hydroxyprolin, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)-pyrrolidin, 2-Hydroxymethylpyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 4-Amino-3-hydroxypyrrolidin, 3-Hydroxy-4-(2-hydroxyethyl)aminopyrrolidin, 3-Acetamidopyrrolidin, 3-(Methylsulfonylamino)-pyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Diazepan, N-Methyldiazepan, N-(2-Hydroxyethyl)-diazepan ausgewählt ist.

20. Verbindung nach Anspruch 19, wobei R₅ und R₆ einen Heterocyclus bilden, der unter Pyrrolidin, 3-Hydroxypyrrolidin, 3-Aminopyrrolidin, 3-Acetamidopyrrolidin, 3-(Methylsulfonylamino)-pyrrolidin, Prolin, 3-Hydroxyprolin, Piperidin, Hydroxypiperidinen, Homopiperidin, Diazepan, N-Methyldiazepan, N-β-Hydroxyethyldiazepan und deren Additionssalzen ausgewählt ist.

21. Verbindung nach Anspruch 16, die ausgewählt ist unter:
- 6-Methoxy-2-pyrrolidin-1-yl-pyridin-3-ylamin;
- 2-(2,5-Dimethyl-pyrrolidin-1-yl)-6-methoxypyridin-3-ylamin;
- 6-Methoxy-2-morpholin-4-yl-pyridin-3-ylamin;
- N-[1-(3-Amino-6-methoxy-pyridin-2-yl)-pyrrolidin-3-yl]-acetamid;
- 6'-Methoxy-3,4,5,6-tetrahydro-2H-[1,2']bipyridinyl-3'-ylamin;
- 2-(3'-Amino-6'-methoxy-3,4,5,6-tetrahydro-2H-[1,2']bipyridinyl-2-yl)-ethanol;
- 6-Methoxy-2-(4-methyl-piperazin-1-yl)-pyridin-3-ylamin;
- 6'-Methoxy-2-methyl-3,4,5,6-tetrahydro-2H-[1,2']bipyridinyl-3'-ylamin;
- 6'-Methoxy-3-methyl-3,4,5,6-tetrahydro-2H-[1,2']bipyridinyl-3'-ylamin;
- 2-(3'-Amino-6'-methoxy-3,4,5,6-tetrahydro-2H[1,2']bipyridinyl-2-yl)-ethanol
sowie deren Additionssalzen.
